(19) Europäisches Patentamt
European Patent Office
Office européen des brevets

(11) EP 3 488 768 A1

(12) EUROPEAN PATENT APPLICATION

(43) Date of publication:
29.05.2019 Bulletin 2019/22

(51) Int Cl.:
A61B 5/00 (2006.01)
A61B 5/11 (2006.01)
G01G 19/44 (2006.01)
A61B 5/024 (2006.01)
A61B 5/0408 (2006.01)
A61B 5/02 (2006.01)
A61B 5/22 (2006.01)
G16H 50/30 (2018.01)
A61B 5/0245 (2006.01)

(21) Application number: 17203696.4

(22) Date of filing: 27.11.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: National Defense Medical Center
Taipei City 114 (TW)

(72) Inventor: CHU, Chi Ming
114 TAIPEI CITY (TW)

(74) Representative: Biggi, Cristina
Bugnion S.p.A.
Viale Lancetti 17
20158 Milano (IT)

(54) **METHOD AND APPARATUS FOR DETERMINING PHYSICAL FITNESS BY MONITORING CARDIAC ACTIVITY**

(57) A method for determining physical fitness by monitoring cardiac activity during exercise is disclosed. The method comprises: measuring a weight, a heart rate and an acceleration of the user of a user to determine a cardiac force index (CFI) of the user, wherein the CFI is calculated with the following equation: CFI=weight × acceleration / heart rate; identifying a maximum CFI at a first time to determine a cardiac force ratio (CFR'), wherein the CFR' is calculated with the following equation: CFR'=CFI/maximum CFI; and determining whether the CFR' at a second time is smaller than a threshold value.

FIG. 4

EP 3 488 768 A1

**Description**

**TECHNICAL FIELD**

The present disclosure generally relates to a method and apparatus for determining physical fitness by monitoring cardiac activity. More particularly, the present disclosure relates to a method and apparatus for determining physical fitness by monitoring the cardiac activity of an individual during exercise using a cardiac parameter derived from a heart rate, a weight and/or an acceleration of the individual.

**BACKGROUND**

Sudden cardiac death is defined as non-violent and unexpected death due to cardiac causes in persons with or without cardiac disease. Sudden cardiac death generally occurs within one hour of the onset of symptoms, if any.
Conventional devices for monitoring cardiac activity during exercise (e.g., heart rate monitors and fitness trackers) can only measure the heart rate, blood oxygen level, blood pressure and/or respiration rate of the user, but these devices cannot provide instantaneous information about the user's cardiac status and cardiac loading condition, which are crucial indicators for evaluating the risk of sudden cardiac death.
Therefore, there is a need for instantaneous monitoring of an individual's cardiac activity to predict or prevent the sudden cardiac death of the individual during exercise.

**SUMMARY**

In some embodiments, a method for determining physical fitness by monitoring cardiac activity during exercise is disclosed. The method comprises: measuring a weight, a heart rate and an acceleration of a user to determine a cardiac force index (CFI) of the user, wherein the CFI is calculated with the following equation: CFI=weight $\times$ acceleration / heart rate; identifying a maximum CFI at a first time to determine a cardiac force ratio (CFR'), wherein the CFR' is calculated with the following equation: CFR'=CFI/maximum CFI; and determining whether the CFR' at a second time is smaller than a threshold value.
In some embodiments, a method for determining physical fitness by monitoring cardiac activity during exercise is disclosed. The method comprises: measuring a weight, a heart rate and an acceleration of a user to determine a cardiac force index (CFI) of the user, wherein the CFI is calculated with the following equation: CFI=weight $\times$ acceleration / heart rate; identifying a maximum CFI to determine a cardiac force ratio (CFR'), wherein the CFR' is calculated with the following equation: CFR'=CFI/maximum CFI; and determining whether the CFR' is smaller than a threshold value for a predetermined period of time.
In some embodiments, an apparatus for determining physical fitness by monitoring cardiac activity during exercise is disclosed. The apparatus comprises: a heart rate measuring device for measuring a heart rate of a user; an acceleration measuring device for measuring an acceleration of the user; and a processor for deriving a cardiac force index (CFI) of the user from the acceleration and the heart rate of the user; wherein the processor is further configured to: identify a maximum CFI at a first time to determine a cardiac force ratio (CFR'), wherein the CFR' is calculated with the following equation: CFR'=CFI/maximum CFI; and determine whether the CFR' at a second time is smaller than a threshold value.

**BRIEF DESCRIPTION OF THE DRAWINGS**

Aspects of the present disclosure are best understood from the following detailed description when read with the accompanying figures. It is noted that various features may not be drawn to scale, and the dimensions of the various features may be arbitrarily increased or reduced for clarity of discussion.

FIG. 1 illustrates a cardiac force ratio (CFR) graph according to one embodiment of the present disclosure.

FIG. 2 illustrates a CFR' graph according to the above embodiment of the present disclosure.

FIG. 3 illustrates a CFR' graph according to one embodiment of the present disclosure.

FIG. 4 illustrates a CFR' graph according to one embodiment of the present disclosure.

FIG. 5 illustrates a CFR' graph according to one embodiment of the present disclosure.

FIG. 6 illustrates a monitoring apparatus for monitoring cardiac activity according to one embodiment of the present disclosure.

## DETAILED DESCRIPTION

Manufacturing and use of at least some embodiments of the present disclosure are discussed in detail below. It should be appreciated, however, that at least some embodiments set forth can deviate from the specific examples provided herein such that they can be implemented in a wide variety of specific contexts. It is to be understood that the following disclosure provides many different embodiments or examples of implementing different features of various embodiments. Specific examples of components and configurations are described below for purposes of discussion. These are, of course, merely examples and are not intended to be limiting.

Some embodiments, or examples, illustrated in the figures are disclosed below using specific language. It will nevertheless be understood that the embodiments and examples are not intended to be limiting. Any alterations and modifications of some of the disclosed embodiments, and any further applications of the principles disclosed in this document, as would normally occur to one of ordinary skill in the pertinent art, fall within the scope of this disclosure.

In addition, the present disclosure may repeat reference numerals and/or letters in the various examples described herein. This repetition is for the purpose of simplicity and clarity and does not in itself dictate a relationship between the various embodiments and/or configurations discussed.

Further, spatially relative terms, such as "beneath," "below," "lower," "above," "upper" and the like, may be used herein for ease of description to describe one element or feature's relationship to another element or feature as illustrated in the figures. The spatially relative terms are intended to encompass different orientations of the device in use or operation in addition to the orientation depicted in the figures. The apparatus may be otherwise oriented (e.g., rotated 90 degrees or at other orientations) and the spatially relative descriptors used herein may be interpreted accordingly.

In one embodiment, the present disclosure provides a cardiac force index (CFI) to reflect the instantaneous cardiac status of an individual. The CFI is associated with the heart rate, acceleration and/or weight of the individual. The CFI indicates the force per heartbeat of the individual. Since the force that acts on the individual can be calculated with Newton's second law of motion (F=ma), the individual's CFI (the force per heartbeat of the individual) can be calculated with the following equation:

$$\text{Cardiac Force Index (CFI)} = \text{Weight} \times \text{Acceleration} / \text{Heart Rate} \qquad (1)$$

The heart rate is the speed of the heartbeat measured by the number of contractions of the heart per minute. Measurement of the heart rate can be carried out by detecting the heart beating or artery pulsing of the individual in any feasible way, such as detecting the blood flow in a capillary vessel of a fingertip or an earlobe, detecting an electrocardiographic signal of a palm area, or detecting an electrocardiographic signal of a chest area with electrodes. The acceleration of the individual during exercise can be measured by an accelerometer of any form, such as a three-axis accelerometer, a nine-axis accelerometer, a sixteen-axis accelerometer, and a G-sensor.

According to one embodiment of the present disclosure, a cardiac force ratio (CFR) that compares the CFI of an individual during exercise to the CFI of a walking individual is provided. The CFR of this embodiment can be calculated with the following equation:

$$\text{Cardio Force Ratio (CFR)} = \text{CFI of Exercise} / \text{CFI of Walking} \qquad (2)$$

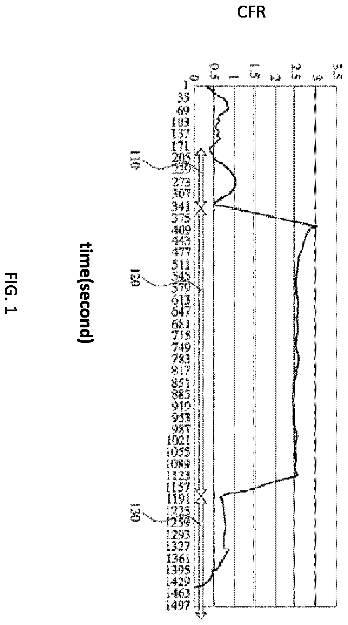

Referring to the drawings, FIG. 1 illustrates a CFR versus time graph according to one embodiment of the present disclosure. In FIG. 1, the CFI of an individual is measured for a time period comprising a pre-exercise phase 110, an exercise phase 120 and a post-exercise phase 130 (the signals before the start of the pre-exercise phase 110 are noises). The pre-exercise phase 110 can be a walking phase in which the individual is walking. The maximum CFI of the pre-exercise phase 110 (which occurs at around the 273th second) is employed as a reference for calculation of cardiac force ratios. That is, the CFR at around the 273th second is set to "1." Upon entering the exercise phase 120 that follows the pre-exercise phase 110, the individual changes from a relatively stable condition of walking into a dynamic condition of exercise, which is indicated by a sharp increase in the acceleration of the individual. Accordingly, the cardiac force ratio quickly rises to a peak. The exercise may comprise running, jogging, swimming, cycling and so on. The peak indicates the explosive power of the individual. In FIG. 1, the peak has a cardiac force ratio of around 3.

After the peak, the cardiac force ratio decreases. The cardiac force ratio decreases most significantly within 40 to 80 seconds after the peak. In one embodiment, the cardiac force ratio decreases most significantly within 70 seconds after the peak. After a lapse of time, the cardiac force ratio becomes stable and enters an endurance period, where the cardiac force ratio is around 2.5. The exercise phase 120 is followed by the post-exercise phase 130 at the 1191th second. FIG. 1 shows that the cardiac force ratio plummets, and this is because the dynamic exercise condition of the individual changes to a relatively static condition.

According to one embodiment of the present disclosure, another cardiac force ratio (CFR') using the maximum CFI of the exercise phase 120 as a reference is provided. The CFR' of this embodiment can be calculated with the following equation:

$$CFR' = CFI/\text{maximum CFI of the exercise phase} \qquad (3)$$

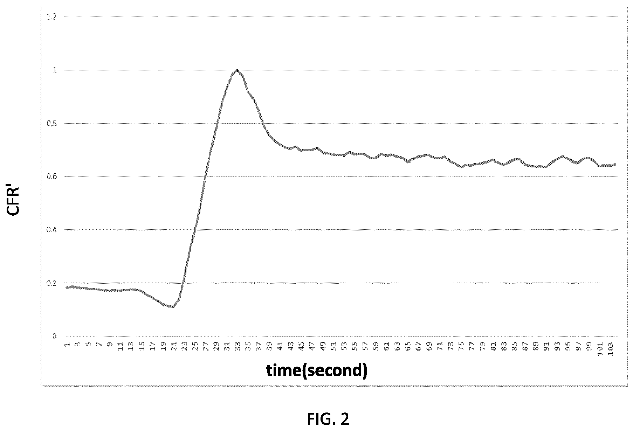

For example, the CFI value for each second of the exercise phase 120 is divided by the maximum CFI of the exercise phase 120 to obtain a CFR'. A CFR' versus time graph can be plotted using the CFR' for each second of the exercise phase 120. FIG. 2 illustrates a CFR' versus time graph according to the above embodiment of the present disclosure. According to FIG. 2, the pre-exercise phase 110 ends at around the 21st second, where the exercise phase 120 begins. The maximum CFI of the exercise phase 120 (which occurs at around the 33th second) is employed as a reference for calculation of the CFR's, and therefore the CFR' at around the 33th second is set to "1." The CFR's decrease at the maximum CFI (the 33th second). The cardiac force ratio decreases most significantly within 40 to 80 seconds after the peak (maximum CFI of the exercise phase 120). In one embodiment, the cardiac force ratio decreases most significantly within 70 seconds after the peak.

As would be discussed below, the CFR' (CFI/maximum CFI of the exercise phase) can provide crucial information for evaluating the risk of sudden cardiac death during exercise.

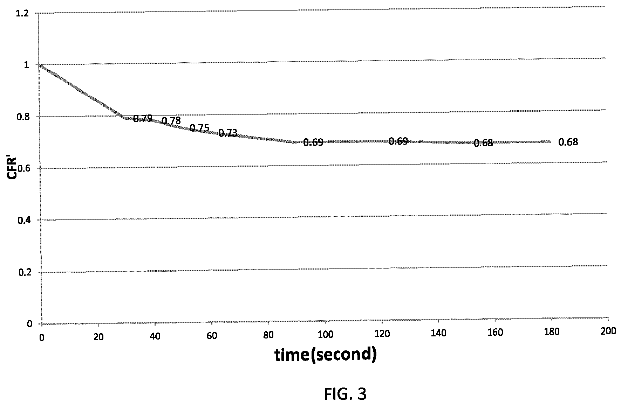

FIG. 3 illustrates a CFR' versus time graph for an individual with normal cardiac condition, wherein the maximum CFI of the exercise phase 120 is set at the 0th second (with the CFR' being "1"). The CFR' at the 30th second (i.e., the CFI at the 30th second divided by the maximum CFI of the exercise phase 120) is 0.79, the CFR' at the 40th second is 0.78, the CFR' at the 50th second is 0.75, the CFR' at the 60th second is 0.73, the CFR' at the 90th second is 0.69, the CFR' at the 120th second is 0.69, the CFR' at the 150th second is 0.68, and the CFR' at the 180th second is 0.68.

It is found that the drop of the CFR' to a value smaller than a threshold value (e.g., between about 0.5 and about 0.7) within about 80 seconds after the maximum CFI of the exercise phase 120 indicates an abnormal cardiac condition where the risk of sudden cardiac death is much higher than normal cardiac condition. In one embodiment, the threshold value of the CFR' is about 0.6.

Additionally, the CFR' can be employed as an indicator for evaluating whether an individual is in good physical fitness. For example, the sudden drop of the CFR' to about 0.6 during exercise may indicates that the individual might not in a good physical fitness.

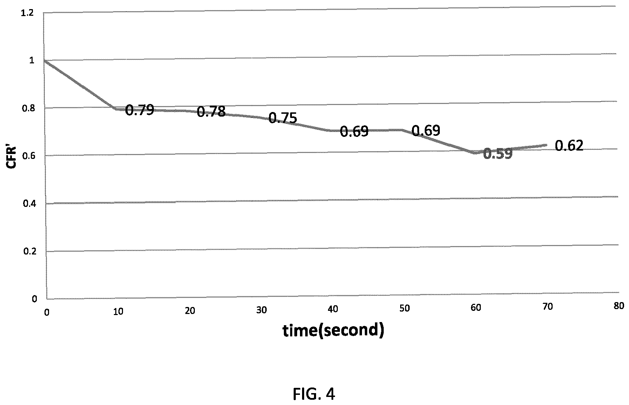

In light of the above, a method for determining physical fitness by monitoring cardiac activity during exercise can be provided. The method comprises measuring the CFI of an individual wherein the CFI for each second in the exercise phase 120 is recorded. The second step of the method is to identify the maximum CFI that occurs at the start of the exercise phase 120. Once the maximum CFI is determined, the CFR' for each second following the maximum CFI of the exercise phase 120 can be calculated. The method then determines whether the CFR' within a period of time after the maximum CFI of the exercise phase 120 is smaller than a threshold value (e.g., 0.6). This period of time may be about 90 seconds following the maximum CFI of the exercise phase 120. This period of time may be about 80 seconds following the maximum CFI of the exercise phase 120. This period of time may be about 70 seconds following the maximum CFI of the exercise phase 120. This period of time may be about 60 seconds following the maximum CFI of the exercise phase 120. This period of time may be about 50 seconds following the maximum CFI of the exercise phase 120. The method further comprises producing an alarm signal in response to detecting that the CFR' within this period of time is smaller than a threshold value (e.g., 0.6). This method for monitoring cardiac activity during exercise can be further illustrated by the embodiment shown in FIG. 4.

FIG. 4 illustrates a CFR' versus time graph for an individual (not all the CFR' values are listed). The identified maximum CFI of the exercise phase 120 is set at the 0th second and is used for calculating the CFR' within a period of time. In this embodiment, the period of time is 70 seconds following the maximum CFI of the exercise phase 120. As shown in FIG. 4, the CFR' at the 10th second (i.e., the CFI at the 10th second divided by the maximum CFI of the exercise phase 120) is 0.79, the CFR' at the 20th second is 0.78, the CFR' at the 30th second is 0.75, the CFR' at the 40th second is 0.69, the CFR' at the 50th second is 0.69, the CFR' at the 60th second is 0.59, and the CFR' at the 70th second is 0.62. Upon detecting that the CFR' at the 60th second is 0.59, which is smaller than the threshold value of 0.6, an alarm signal would be produced.

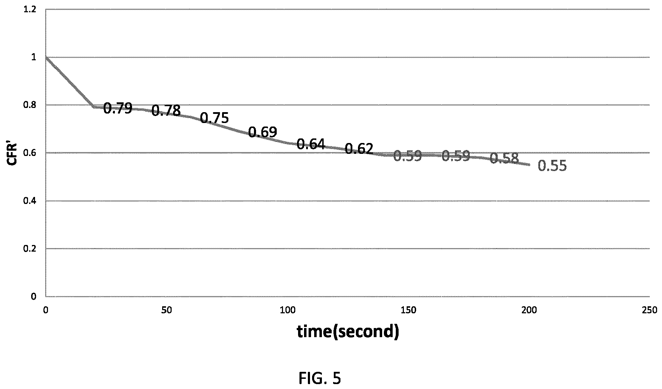

According to another embodiment of the present disclosure, a method for determining physical fitness by monitoring cardiac activity during exercise is provided. The method comprises: measuring the CFI of an individual; identifying the maximum CFI that occurs at the start of the exercise phase 120; and determines whether the CFR' after the maximum CFI of the exercise phase 120 is smaller than a threshold value (e.g., 0.6) for a period of time. This period of time may be about 30, 40, 50, 60, 70, 80, 90, 100, 110 or 120 seconds. For example, the method may comprise determining whether the CFR' after the maximum CFI of the exercise phase 120 is smaller than 0.6 for 60 seconds. This method for monitoring cardiac activity during exercise can be further illustrated by the embodiment shown in FIG. 5.

FIG. 5 illustrates a CFR' versus time graph for an individual (not all the CFR' values are listed). The identified maximum CFI of the exercise phase 120 is set at the $0^{th}$ second. As shown in FIG. 5, the CFR' at the $20^{th}$ second is 0.79, the CFR' at the $40^{th}$ second is 0.78, the CFR' at the $60^{th}$ second is 0.75, the CFR' at the $80^{th}$ second is 0.69, the CFR' at the $100^{th}$ second is 0.64, the CFR' at the $120^{th}$ second is 0.62, the CFR' at the $140^{th}$ second is 0.59, the CFR' at the $160^{th}$ second is 0.59, the CFR' at the $180^{th}$ second is 0.58, and the CFR' at the $200^{th}$ second is 0.55. Upon detecting that the CFR' is smaller than the threshold value of 0.6 for at least 60 seconds (from the $140^{th}$ second with a CFR' of 0.59 to the $200^{th}$ second with a CFR' of 0.55), an alarm signal would be produced.

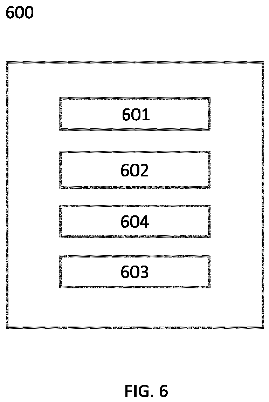

FIG. 6 illustrates a monitoring apparatus 600 for determining physical fitness by monitoring a user's cardiac activity during exercise. The monitoring apparatus 600 comprises a heart rate measuring device 601 for measuring a heart rate of the user. The monitoring apparatus 600 further comprises an acceleration measuring device 602 for measuring an acceleration of the user. The acceleration measuring device 602 may be a three-axis accelerometer, a nine-axis accelerometer, a sixteen-axis accelerometer, or a G-sensor. The monitoring apparatus 600 comprises a processor 603 for deriving the CFI from the acceleration and the heart rate of the user. The processor is also configured to identify the maximum CFI that occurs at the start of an exercise phase of the user and determine whether any CFR' value within about 70 seconds after the maximum CFI of the exercise phase is smaller than a threshold value (e.g., 0.6). The monitoring apparatus 600 further comprises an alarming device 604 for generating an alarm signal in response to the processor 603 determining that at least one CFR' value within about 70 seconds after the maximum CFI of the exercise phase is smaller than 0.6.

In one embodiment, the processor is configured to determine whether the CFR' after the maximum CFI of the exercise phase is smaller than 0.6 for a period of time (e.g., 60 seconds), and the alarming device 604 generates an alarm signal in response to the processor 603 determining that the CFR' is smaller than the threshold value of 0.6 for at least 60 seconds.

As used herein, the singular terms "a," "an," and "the" may include plural referents unless the context clearly dictates otherwise. In the description of some embodiments, a component provided or disposed "on" or "over" another component can encompass cases where the former component is directly on (e.g., in physical or direct contact with) the latter component, as well as cases where one or more intervening components are located between the former component and the latter component.

Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified.

As used herein, the terms "approximately," "substantially," "substantial," "around" and "about" are used to describe and account for small variations. When used in conjunction with an event or circumstance, the terms can refer to instances in which the event or circumstance occurs precisely as well as instances in which the event or circumstance occurs to a close approximation.

While the present disclosure has been described and illustrated with reference to specific embodiments thereof, these descriptions and illustrations do not limit the present disclosure. It should be understood by those skilled in the art that various changes may be made and equivalents may be substituted without departing from the true spirit and scope of the present disclosure as defined by the appended claims. The illustrations may not be necessarily drawn to scale. There may be distinctions between the artistic renditions in the present disclosure and the actual apparatus due to manufacturing processes and tolerances.

**Claims**

1.  A method for determining physical fitness by monitoring cardiac activity during exercise, comprising:

    measuring a weight, a heart rate and an acceleration of a user to determine a cardiac force index (CFI) of the user, wherein the CFI is derived from the following equation:

$$CFI = weight \times acceleration / heart\ rate;$$

identifying a maximum CFI at a first time to determine a cardiac force ratio (CFR'), wherein the CFR' is derived from the following equation:

$$CFR' = CFI/maximum\ CFI;$$

and
determining whether the CFR' at a second time is smaller than a threshold value.

2. The method of claim 1, wherein the threshold value is between about 0.5 and about 0.7.

3. The method of claim 1, wherein the CFI is measured for a first period of time that comprises an exercise phase in which the user is doing exercise.

4. The method of claim 3, wherein the first time and the second time both fall within the exercise phase.

5. The method of claim 1, wherein the second time is less than about 40 to 80 seconds after the first time.

6. A method for determining physical fitness by monitoring cardiac activity during exercise, comprising:

measuring a weight, a heart rate and an acceleration of a user to determine a cardiac force index (CFI) of the user, wherein the CFI is derived from the following equation:

$$CFI = weight \times acceleration / heart\ rate;$$

identifying a maximum CFI to determine a cardiac force ratio (CFR'), wherein the CFR' is derived from the following equation:

$$CFR' = CFI/maximum\ CFI;$$

and
determining whether the CFR' is smaller than a threshold value for a predetermined period of time.

7. The method of claim 6, wherein the predetermined period of time is between about 30 seconds and about 120 seconds.

8. The method of claim 6, wherein the threshold value is about 0.6.

9. An apparatus for determining physical fitness by monitoring cardiac activity during exercise, comprising:

a heart rate measuring device for measuring a heart rate of a user;
an acceleration measuring device for measuring an acceleration of the user; and
a processor for deriving a cardiac force index (CFI) of the user from the acceleration and the heart rate of the user;
wherein the processor is further configured to:

identify a maximum CFI at a first time to determine a cardiac force ratio (CFR'), wherein the CFR' is calculated with the following equation:

$$CFR' = CFI/maximum\ CFI$$

determine whether the CFR' at a second time is smaller than a threshold value.

**10.** The apparatus of claim 9, further comprising a weighing device for measuring a weight of the user.

**11.** The apparatus of claim 10, wherein the CFI is calculated with the following equation:

$$CFI = weight \times acceleration / heart\ rate$$

**12.** The apparatus of claim 9, wherein the threshold value is between about 0.3 and about 0.8.

**13.** The apparatus of claim 9, further comprising an alarming device for generating an alarm signal in response to the processor determining that the CFR' at the second time is smaller than the threshold value.

**14.** The apparatus of claim 9, wherein the second time occurs less than about 70 seconds after the first time.

**15.** The apparatus of claim 9, wherein the first time and the second time both fall within an exercise phase in which the user is doing an exercise.

FIG. 1

FIG. 2

FIG. 3

FIG. 4

FIG. 5

600

FIG. 6

Europäisches
Patentamt

European
Patent Office

Office européen
des brevets

**EUROPEAN SEARCH REPORT**

Application Number

EP 17 20 3696

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/058314 A1 (CHU CHI MING [TW]) 3 March 2016 (2016-03-03) * paragraphs [0023], [0024], [0028], [0031], [0035], [0061], [0067] * * figures 1, 2 * ----- | 1-15 | INV. A61B5/00 A61B5/02 A61B5/11 A61B5/22 G01G19/44 G16H50/30 |
| A | WO 2016/044831 A1 (ATHLETE ARCHITECT LLC [US]) 24 March 2016 (2016-03-24) * paragraphs [0028], [0065], [0165], [0166], [0170] * * figures 10, 11 * ----- | 1-15 | ADD. A61B5/024 A61B5/0245 A61B5/0408 |
| A | US 2017/216673 A1 (ARMSTRONG JUDD [AU] ET AL) 3 August 2017 (2017-08-03) * the whole document * ----- | 1-15 | |
| A | US 2016/051156 A1 (KIM HEE CHAN [KR] ET AL) 25 February 2016 (2016-02-25) * the whole document * ----- | 1-15 | |

TECHNICAL FIELDS
SEARCHED       (IPC)

A61B
G01G
G16H

The present search report has been drawn up for all claims

| Place of search | Date of completion of the search | Examiner |
|---|---|---|
| The Hague | 2 May 2018 | Meyer, Wolfgang |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

  .................................................................................

& : member of the same patent family, corresponding
    document

EPO FORM 1503 03.82 (P04C01)

**EP 3 488 768 A1**

## ANNEX TO THE EUROPEAN SEARCH REPORT
## ON EUROPEAN PATENT APPLICATION NO.

EP 17 20 3696

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2018

| Patent document cited in search report | | Publication date | Patent family member(s) | | Publication date |
|---|---|---|---|---|---|
| US 2016058314 | A1 | 03-03-2016 | TW | 201609051 A | 16-03-2016 |
| | | | US | 2016058314 A1 | 03-03-2016 |
| WO 2016044831 | A1 | 24-03-2016 | EP | 3194890 A1 | 26-07-2017 |
| | | | US | 2017189752 A1 | 06-07-2017 |
| | | | US | 2018043211 A1 | 15-02-2018 |
| | | | WO | 2016044831 A1 | 24-03-2016 |
| US 2017216673 | A1 | 03-08-2017 | NONE | | |
| US 2016051156 | A1 | 25-02-2016 | US | 2016051156 A1 | 25-02-2016 |
| | | | WO | 2014157896 A1 | 02-10-2014 |

EPO FORM P0459

For more details about this annex : see Official Journal of the European Patent Office, No. 12/82